# EUROPEAN PATENT APPLICATION

(11) **EP 3 557 586 A1**
(43) Date of publication of application: **23.10.2019**
(21) Application number: 18168572.8
(22) Date of filing: 20.04.2018
(51) Int. Cl.: G16H 20/30

(54) **METHOD AND APPARATUS FOR REDUCING AN EFFECT OF PULSATING LIGHT ON A SUBJECT**

(71) Applicant: Koninklijke Philips N.V., 5656 AE Eindhoven (NL)
(72) Inventor: AARTS, Ronaldus Maria, 5656 AE Eindhoven (NL); VAN EE, Raymond, 5656 AE Eindhoven (NL); TEN KATE, Warner Rudolph Theophile, 5656 AE Eindhoven (NL); JOHNSON, Mark Thomas, 5656 AE Eindhoven (NL)
(74) Representative: de Haan, Poul Erik

(57) **Abstract**

According to an aspect, there is provided an apparatus for compensating pulsating light, the apparatus comprising: a processing unit configured to receive, from a light sensor, a signal indicative of light incident on a subject; process the received signal to determine if the incident light is pulsating; determine one or more parameters for light that is to be emitted between pulses in the incident light to reduce the overall temporal variation of the light incident on the subject if it is determined that the incident light is pulsating; and control one or more light sources to emit light between the pulses in the incident light according to the determined one or more parameters.

## Description

### FIELD OF THE INVENTION

This disclosure relates to a method and apparatus for reducing an effect of pulsating light on a subject, for example reducing the risk of an epileptic seizure for a subject that has epilepsy.

### BACKGROUND OF THE INVENTION

Epilepsy is a heterogeneous family of chronic disorders with highly variable clinical features, patient histories, and patterns of treatment response. Epilepsy is characterised by episodes of neurologic dysfunction such as seizures. Anticonvulsant medications can be administered to many people that have epilepsy for preventing seizures, but they often suffer side effects. For 20-40% of people with epilepsy, medications are not effective, and even after surgical removal of epilepsy-causing brain tissue, many of them continue to experience spontaneous seizures. Despite the fact that seizures occur infrequently, people with epilepsy often experience persistent anxiety due to the possibility of a seizure occurring. Clinicians who care for people with epilepsy have long known that many people are aware of periods when seizures are more likely, though they can rarely specify an exact time when seizures will happen.

Epilepsy seizures bring, in addition to the discomfort and hazard incurred during their progression, further negative after effects that impact significantly health and quality of life. A first well-known example is that epileptic seizures can make people with epilepsy more prone to falls and injuries. In the general population of people with epilepsy, it is considered that about 30% of people with epilepsy eventually develop clinical depression.

Epilepsy in elderly people poses several additional problems including: diagnostic difficulties; susceptibility to anti-epileptic drug (AED) side-effects and toxicity; increased likelihood of interaction between AEDs with other medication necessary for treating other existing chronic conditions; social difficulties and physical restrictions to lifestyle leading to increased isolation; increased impact of driving restrictions leading to dependency on others to enable them to attend consultations and therapy sessions (which implies that elderly people with epilepsy are often less adherent to their treatment plan); seizures that cause falls are more likely to cause serious injury in older people that can, in aftermath, given the already fragile health of these people, lead to death; the requirement for multidisciplinary services in the community, including liaison nurses, social workers and occupational therapists.

Given the significant impact on the health and quality of life of people with epilepsy, as well as the complications related to seizure treatment, there is a need for solutions that can reduce the risk of (or even prevent) seizures occurring.

### SUMMARY OF THE INVENTION

For some people with epilepsy, seizures can be triggered by pulsating (e.g. flashing or flickering) light in the environment of the subject (e.g. from television/movie pictures, computer games, ambient lighting, strobe lighting, light appearing to flash through gaps between trees alongside a road while driving, during theatre or music shows, etc.). Even for people that do not suffer from epilepsy, pulsating light can be distracting or annoying, particularly if they are trying to concentrate, e.g. when driving. , The invention aims to compensate pulsating light (for example to reduce the risk or occurrence of epileptic seizures) by detecting pulsating light that may trigger an epileptic seizure and emitting light between the pulses in the incident light to reduce the visibility or perception of that pulsating light to the subject.

According to a first aspect, there is provided an apparatus for compensating pulsating light, the apparatus comprising a processing unit configured to: receive, from a light sensor, a signal indicative of light incident on the subject; process the received signal to determine if the incident light is pulsating; determine one or more parameters for light that is to be emitted between pulses in the incident light to reduce the overall temporal variation of the light incident on the subject if it is determined that the incident light is pulsating; and control one or more light sources to emit light between the pulses in the incident light according to the determined one or more parameters. Thus, the apparatus operates to control light source(s) to emit light to compensate the pulsating light and reduce the visibility or perception of pulsating light to the subject, thereby reducing the risk of an epileptic seizure, and/or reducing the annoyance or distraction caused by the pulsating light.

In some embodiments, the one or more parameters comprises a pulsation frequency and phase of the light to be emitted between the pulses in the incident light, and wherein the processing unit is configured to implement a phase locked loop, PLL, to process the received signal and to determine the pulsation frequency and phase of light that is to be emitted between the pulses in the incident light, wherein the received signal of the light incident on the subject is input to the PLL, and the PLL outputs a control signal for the one or more light sources having a pulsation frequency that matches or substantially matches a pulsation frequency of the incident light that is pulsating, and a phase that is opposite to the phase of the incident light that is pulsating. The use of a PLL is beneficial as the light source(s) can be controlled to provide a relatively constant overall light intensity for the subject.

In some embodiments, the one or more parameters comprises a pulsation frequency and phase of the light to be emitted between the pulses in the incident light, and wherein the processing unit is configured to determine the one or more parameters by estimating a pulsation frequency and phase of the incident light that is pulsating; and determining the pulsation frequency and phase of light that is to be emitted between the pulses in the incident light such that the frequency of the emitted light matches the estimated pulsation frequency of the incident light that is determined to be pulsating, and determining the phase of the light that is to be emitted between the pulses in the incident light to be opposite to the phase of the incident light that is pulsating. This embodiment is advantageous where the pulsation frequency is changing over time as the frequency of the light to be emitted can readily be adapted to that changing frequency.

In some embodiments, the one or more parameters comprises one or more of a colour of the light, an intensity of the light, a pulsation frequency of the light, and a duration of each pulse of the light.

In some embodiments, the processing unit is configured to implement a feedback loop to process the received signal and to determine values of the one or more parameters of light that is to be emitted between the pulses in the incident light based on values of the one or more parameters for the light incident on the subject that are pulsating. The use of a feedback loop is beneficial as the light source(s) can be controlled to provide a relatively constant overall light intensity for the subject.

In these embodiments, the feedback loop can determine values for the one or more parameters for the light to be emitted between the pulses in the incident light such that values of the one or more parameters for light subsequently incident on the subject are substantially constant over time.

In some embodiments, the processing unit is configured to determine a colour of the light to be emitted between the pulses in the incident light to match or substantially match the colour of a component of the incident light that is determined to be pulsating. This embodiment can be useful where the change in intensity of the ambient light is relatively small, but the colour of the ambient light is pulsating (i.e. there may be a flashing red light component in the ambient light). In this case, these embodiments emit light of the same colour as the pulsating light (e.g. red in this example) to reduce the perception of the flashing red component.

In some embodiments, the processing unit is configured to determine an intensity of the light to be emitted between the pulses in the incident light to match or substantially match the intensity of a component of the incident light that is determined to be pulsating. This embodiment provides that the overall intensity of the light incident on the subject can be made relatively constant.

In some embodiments, the processing unit is configured to determine a pulsation frequency of the light to be emitted between the pulses in the incident light such that the light to be emitted has the opposite phase to the phase of a component of the incident light that is determined to be pulsating. This embodiment provides that the emitted light will 'fill the gaps' between the pulsations in the incident light.

In some embodiments, the processing unit is configured to determine a duration of each pulse of light to be emitted between the pulses in the incident light based on a time between consecutive pulses of a component of the incident light that is determined to be pulsating. This embodiment provides that the emitted light will 'fill the gaps' between the pulsations in the incident light.

In some embodiments, the received signal is a signal indicative of the intensity of the light incident on the subject.

In some embodiments, the processing unit is configured to determine if the incident light is pulsating at the same frequency or a similar frequency as brain oscillations of the subject. This embodiment is useful as the light source(s) may only be used to emit light when the incident light is pulsating at a frequency that might affect the brain of the subject and trigger an epileptic seizure.

In some embodiments, the apparatus further comprises the light sensor and/or one or more light sources.

In some embodiments, the light sensor is arranged in the apparatus such that the light sensor is close to an eye of the subject when the apparatus is in use by the subject. This has the advantage that the light sensor can measure the incident light as it is perceived by the subject.

In some embodiments, the one or more light sources are arranged in the apparatus such that the one or more light sources are visible to the subject when the apparatus is in use by the subject.

In some embodiments, the apparatus is in the form of spectacles or other eye or head wear. These embodiments provide that the apparatus can be relatively unobtrusive and easy for the subject to use.

According to a second aspect, there is provided a computer-implemented method of compensating pulsating light, the method comprising receiving, from a light sensor, a signal indicative of light incident on a subject; processing the received signal to determine if the incident light is pulsating; determining one or more parameters for light that is to be emitted between pulses in the incident light to reduce the overall temporal variation of the light incident on the subject if it is determined that the incident light is pulsating; and controlling one or more light sources to emit light between the pulses in the incident light according to the determined one or more parameters. Thus, the method operates to control light source(s) to emit light to reduce the visibility or perception of pulsating light to the subject, thereby reducing the risk of an epileptic seizure, and/or reducing the annoyance or distraction caused by the pulsating light.

In some embodiments, the one or more parameters comprises a pulsation frequency and phase of the light to be emitted between the pulses in the incident light, and wherein the steps of processing and determining comprises implementing a phase locked loop, PLL, to process the received signal and to determine the pulsation frequency and phase of light that is to be emitted between the pulses in the incident light, wherein the received signal of the light incident on the subject is input to the PLL, and the PLL outputs a control signal for the one or more light sources having a pulsation frequency that matches or substantially matches a pulsation frequency of the incident light that is pulsating, and a phase that is opposite to the phase of the incident light that is pulsating. The use of a PLL is beneficial as the light source(s) can be controlled to provide a relatively constant overall light intensity for the subject.

In some embodiments, the one or more parameters comprises a pulsation frequency and phase of the light to be emitted between the pulses in the incident light, and wherein the step of determining comprises determining the one or more parameters by estimating a pulsation frequency and phase of the incident light that is pulsating; and determining the pulsation frequency and phase of light that is to be emitted between the pulses in the incident light such that the frequency of the emitted light matches the estimated pulsation frequency of the incident light that is determined to be pulsating, and determining the phase of the light that is to be emitted between the pulses in the incident light to be opposite to the phase of the incident light that is pulsating. This embodiment is useful where the pulsation frequency is changing over time as the frequency of the light to be emitted can readily be adapted to that changing frequency.

In some embodiments, the one or more parameters comprises one or more of a colour of the light, an intensity of the light, a pulsation frequency of the light, and a duration of each pulse of the light.

In some embodiments, the steps of processing and determining comprises implementing a feedback loop to process the received signal and to determine values of the one or more parameters of light that is to be emitted between the pulses in the incident light based on values of the one or more parameters for the light incident on the subject that are pulsating. The use of a feedback loop is beneficial as the light source(s) can be controlled to provide a relatively constant overall light intensity for the subject.

In these embodiments, the feedback loop can determine values for the one or more parameters for the light to be emitted between the pulses in the incident light such that values of the one or more parameters for light subsequently incident on the subject are substantially constant over time.

In some embodiments, the step of determining comprises determining a colour of the light to be emitted between the pulses in the incident light to match or substantially match the colour of a component of the incident light that is determined to be pulsating. This embodiment can be useful where the change in intensity of the ambient light is relatively small, but the colour of the ambient light is pulsating (i.e. there may be a flashing red light component in the ambient light). In this case, these embodiments emit light of the same colour as the pulsating light (e.g. red in this example) to reduce the perception of the flashing red component.

In some embodiments, the step of determining comprises determining an intensity of the light to be emitted between the pulses in the incident light to match or substantially match the intensity of a component of the incident light that is determined to be pulsating. This embodiment provides that the overall intensity of the light incident on the subject can be made relatively constant.

In some embodiments, the step of determining comprises determining a pulsation frequency of the light to be emitted between the pulses in the incident light such that the light to be emitted has the opposite phase to the phase of a component of the incident light that is determined to be pulsating. This embodiment provides that the emitted light will 'fill the gaps' between the pulsations in the incident light.

In some embodiments, the step of determining comprises determining a duration of each pulse of light to be emitted between the pulses in the incident light based on a time between consecutive pulses of a component of the incident light that is determined to be pulsating. This embodiment provides that the emitted light will 'fill the gaps' between the pulsations in the incident light.

In some embodiments, the method further comprises the step of emitting light between the pulses in the incident light according to the determined one or more parameters.

In some embodiments, the received signal is a signal indicative of the intensity of the light incident on the subject.

In some embodiments, the step of processing comprises determining if the incident light is pulsating at the same frequency or a similar frequency as brain oscillations of the subject. This embodiment is useful as the light source(s) may only be used to emit light when the incident light is pulsating at a frequency that might affect the brain of the subject and trigger an epileptic seizure.

According to a third aspect, there is provided a computer program product comprising a computer readable medium having computer readable code embodied therein, the computer readable code being configured such that, on execution by a suitable computer or processor, the computer or processor is caused to perform the method according to the second aspect or any embodiment thereof.

It will be appreciated by those skilled in the art that two or more of the above-mentioned options, implementations, and/or aspects of the invention may be combined in any way deemed useful.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a block diagram of an exemplary apparatus for compensating pulsating light according to the invention;
Fig. 2 is a diagram of an exemplary implementation of an apparatus in the form of spectacles;
Fig. 3 is a flow chart illustrating a method of compensating pulsating light according to an aspect; and
Fig. 4 shows several signals representing light intensity over time.

### DETAILED DESCRIPTION OF EMBODIMENTS

Pulsating visual stimuli can cause steady state visually evoked potentials, that is, brain oscillations with a frequency matching the visual stimulation, and may cause epileptic seizures for people with epilepsy. Even for people without epilepsy, pulsating (e.g. flashing or flickering) light can be distracting or annoying. The invention detects these pulsating stimuli and emits light into or towards the eyes of a subject to decrease the contrast or visibility of these pulsating stimuli (e.g. with the aim of providing a more or less flat envelope in the incident light), hence reducing the probability of an epileptic event, or reducing distraction or annoyance of the pulsating light.

Pulsating light can be generally understood to be any light that is cyclic or rapidly changing, e.g. varying consistently or frequently over time. For example, pulsating light can be light that is generally oscillating or cycling in intensity between a first intensity level and a second intensity level over time. As another example, pulsating light can be light that is generally oscillating or cycling in colour between a first colour and a second colour over time. Other pulsating light can oscillate or cycle in both intensity and colour. The variation/oscillation of the pulsating light can be at a frequency that is visually perceptible to a subject (i.e. a subject can see the pulsation for themselves). For example the frequency of pulsation may be less than, say, 20 Hz (although the frequency at which pulsating light is perceptible is subject-dependent). However, the frequency of pulsation may be at a frequency that is not or tends not to be visually perceptible, or not easily perceptible, to the subject. For example, lighting driven by a mains power supply may pulse at a frequency of 50 Hz or 60 Hz (depending on the frequency of the mains power supply), and this pulsation is typically not perceptible to a subject.

It will be appreciated that although the invention is primarily described below in places with reference to reducing the risk of epileptic seizures in people with epilepsy, the invention can be used to reduce the effect of pulsating light on any person, for example to reduce distraction or annoyance caused by pulsating light (e.g. while trying to concentrate or drive a car).

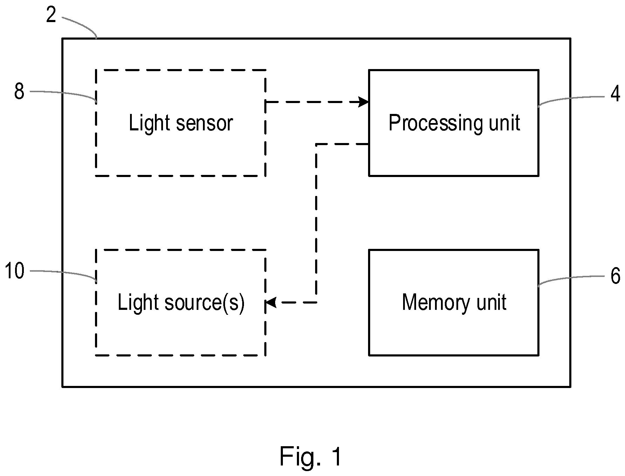

An exemplary apparatus 2 for compensating pulsating light is shown in Fig. 1. The apparatus 2 includes a processing unit 4 that controls the operation of the apparatus 2 and that can be configured to execute or perform the methods described herein. The processing unit 4 can be implemented in numerous ways, with software and/or hardware, to perform the various functions described herein. The processing unit 4 may comprise one or more microprocessors or digital signal processor (DSPs) that may be programmed using software or computer program code to perform the required functions and/or to control components of the processing unit 4 to effect the required functions. The processing unit 4 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), and field-programmable gate arrays (FPGAs).

The processing unit 4 is connected to a memory unit 6 that can store data, information and/or signals for use by the processing unit 4 in controlling the operation of the apparatus 2 and/or in executing or performing the methods described herein. In some implementations the memory unit 6 stores computer-readable code that can be executed by the processing unit 4 so that the processing unit 4 performs one or more functions, including the methods described herein. The memory unit 6 can comprise any type of non-transitory machine-readable medium, such as cache or system memory including volatile and nonvolatile computer memory such as random access memory (RAM) static RAM (SRAM), dynamic RAM (DRAM), read-only memory (ROM), programmable ROM (PROM), erasable PROM (EPROM) and electrically erasable PROM (EEPROM), implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc.

To detect pulsating stimuli, a light sensor is required to provide a signal indicative of light incident on the subject. A light sensor 8 is shown in Fig. 1 as part of the apparatus 2, with the light sensor 8 being connected to the processing unit 4 so that the signal from the light sensor 6 is provided to the processing unit 4 for analysis. The light sensor 8 measures the light over time (for example measuring the light at discrete time points according to a sampling frequency for the light sensor 8) and thus provides a signal in the form of a time series of measurements of the light to the processing unit 4. Preferably the measurements of the light are provided to the processing unit 4 in real-time or near real-time. The light sensor 8 may be any type of light sensor 8 or photosensitive device that can measure light that is incident on the light sensor 8. For example the light sensor 8 can be a photodiode or a photoresistor. The light sensor 8 may measure the intensity of the incident light over time and/or the colour of the incident light over time.

It will be appreciated that in some embodiments the light sensor 8 is in the same housing of the apparatus 2 as the processing unit 4 and the memory unit 6, but in other embodiments the light sensor 8 can be separate from the part of the apparatus 2 that houses the processing unit 4 and the memory unit 6. In alternative embodiments, the light sensor 8 may be completely separate from the apparatus 2 (i.e. it is not part of the apparatus 2), in which case the apparatus 2 or processing unit 4 can comprise interface circuitry (not shown in Fig. 1) that enables the apparatus 2 and processing unit 4 to receive the measurement signal.

In use, the light sensor 8 is positioned so that it measures the light incident on the subject of interest and outputs a corresponding signal. Since an epileptic seizure can be triggered by the subject viewing pulsating stimuli, or annoyance or distraction can be caused by viewing pulsating stimuli, the light sensor 8 is preferably positioned so that it is able to measure the light visible to the subject. For example the light sensor 8 can be positioned close to an eye or the eyes of the subject, to measure light that is incident on an eye or the eyes of the subject.

To reduce the overall temporal variation of the light incident on the subject, and thus reduce the risk of an epileptic seizure or distraction or annoyance for a subject, the processing unit 4 controls one or more light sources 10 to emit light to counteract the pulsating light. This light is to be emitted between the pulses in the pulsating light. One or more light sources 10 are shown in Fig. 1 as part of the apparatus 2, with the light source(s) 10 being connected to the processing unit 4 so that the processing unit 4 can control the light source(s) 10 to emit light, for example in response to a control signal from the processing unit 4. Various parameters of the light emitted by the light source(s) 10 may be controllable by the processing unit 4. For example the intensity (or maximum intensity) of the light emitted by the light source(s) 10 may be controllable, the colour of the light emitted by the light source(s) 10 may be controllable, the light source(s) 10 may be controllable to emit pulsating light, with the frequency of the pulsation being controllable, and/or the duration of each pulse of light from the light source(s) 10 may be controllable.

The light source(s) 10 may be any type or combination of types of light source 10 that can emit light. For example the light source(s) 10 can be one or more light emitting diodes, LEDs, although other types of light source can be used.

In some embodiments, the light source(s) 10 can comprise light sources having different colours (for example red, green and blue LEDs) that can be controlled together according to a control signal from the processing unit 4 to provide light of a desired colour. Alternatively the light source(s) 10 can be controllable by a control signal from the processing unit 4 to provide light of a desired colour.

It will be appreciated that in some embodiments the light source(s) 10 are in the same housing of the apparatus 2 as the processing unit 4 and the memory unit 6, but in other embodiments the light source(s) 10 can be separate from the part of the apparatus 2 that houses the processing unit 4 and the memory unit 6. In alternative embodiments, the light source(s) 10 may be completely separate from the apparatus 2 (i.e. they are not part of the apparatus 2), in which case the apparatus 2 or processing unit 4 can comprise interface circuitry (not shown in Fig. 1) that enables the apparatus 2 and processing unit 4 to provide a suitable control signal to the light source(s) 10.

In use, the light source(s) 10 should be positioned so that it/they emit light that is visible to the subject. In some embodiments, the light source(s) 10 can be positioned directly in the field of view of the subject, for example the light source(s) 10 can be positioned in front of an eye or eyes, which could include close to the eye or eyes, e.g. within a few millimetres (mm) or centimetres (cm), or further away, for example the light source(s) 10 could be located in the environment of the subject within the field of view of the subject. In other embodiments, the light source(s) 10 can be, or be positioned, outside of the field of view of the subject. In this case, a reflecting element could be provided (e.g. the lens or lenses of spectacles) to direct the light towards the eye or eyes of the subject. Alternatively, the light source(s) 10 may be arranged to illuminate a part of the environment around the subject (e.g. a wall, objects in the environment, etc.) or generally illuminate the environment around the subject.

In some embodiments, the light sensor 8 and light source(s) 10 can be arranged on, attached to or part of, a wearable device that can be carried or worn by the subject. For example, the light sensor 8 and light source(s) 10 can be arranged on, attached to or part of a pair of spectacles, or one or more contact lenses, or other device that can be worn on the head or eye(s) of the subject, for example a headband, a nose clip, a necklace, etc.

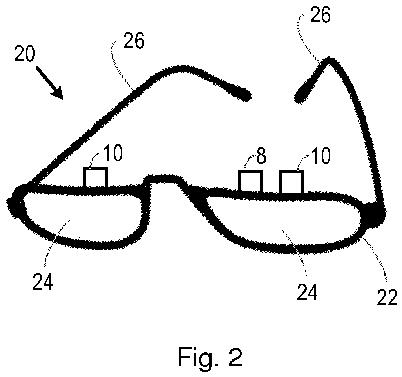

An exemplary implementation of the light sensor 8 and light source(s) 10 on a pair of spectacles 20 is shown in Fig. 2. In Fig. 2, the spectacles 20 comprise a frame 22 for holding or supporting lenses 24 for each of the subject's eyes, and a pair of arms 26 that extend towards the subject's ears to hold the spectacles 20 in place on the subject's head. It will be appreciated that the lenses 24 may be used to correct the vision of the subject in the event that the subject has defects in their vision (e.g. short- or long-sightedness), or the lenses 24 may be optically neutral (e.g. if the subject does not have any, or any substantial, vision defects). In the latter case, the lenses 24 may be omitted if optical correction of the subject's vision is not required. It will also be appreciated that the shape of the frame 22 and arms 26 shown in Fig. 2 is exemplary and other shapes and configurations are possible.

A light sensor 8 is attached to the frame 22. In Fig. 2, the light sensor 8 is attached to (or integral with) the frame 22 near to the part of the frame 22 that rests on the bridge of the nose of the subject when the spectacles 20 are in use. Alternatively the light sensor 8 could be positioned on a different part of the frame 22. In either case, the light sensor 8 is preferably positioned on the frame 22 so that it can measure light incident towards the subject. Fig. 2 also shows two light sources 10 (e.g. LEDs) that are attached to (or integral with) the frame 22 either side of the part of the frame 22 that rests on the bridge of the nose of the subject when the spectacles 20 are in use. In this way, each light source 10 emits light for a respective eye of the subject. Alternatively, a single light source 10 could be provided that is located more centrally on the frame 22. In an alternative arrangement, the light sources 10 could be positioned on or integral with the arms 26 of the spectacles 20. In this arrangement, each light source 10 could be able to emit light directly towards a respective eye of the subject, or each light source 10 could be out of the direct field of view of the subject and instead the light sources 10 could direct light towards a respective lens 24, with each lens 24 reflecting the light towards a respective eye.

It will be noted that Fig. 2 does not show the processing unit 4 or memory unit 6. In some embodiments, as noted above, the processing unit 4 and memory unit 6 can be part of or attached to the spectacles 20 (in which case the spectacles 20 are an exemplary form factor for the apparatus 2). Alternatively, in other embodiments, the processing unit 4 and memory unit 6 (forming the apparatus 2) are located separately from the spectacles 20, and for example the apparatus 2 can be part of a computing device, such as a server, a computer, a laptop, a tablet, a smartphone, etc. In that case, interface circuitry can be provided on the spectacles 20 to enable communication of measurements/signal from the light sensor 8 to the processing unit 4 and communication of a control signal or signals from the processing unit 4 to the light source(s) 10.

Generally, in embodiments where the light sensor 8 and the light source(s) 10 are not integral with the apparatus 2, or not integral with the part of the apparatus 2 comprising the processing unit 4 and the memory unit 6, the apparatus 2 (in the former case) or the part of the apparatus 2 comprising the processing unit 4 and the memory unit 6 (in the latter case) can be any type of electronic device or computing device, such as a server, a computer, a laptop, a tablet, a smartphone, etc.

It will be appreciated that a practical implementation of an apparatus 2 may include additional components to those shown in Fig. 1. For example the apparatus 2 may also include a power supply, such as a battery, or components for enabling the apparatus 2 to be connected to a mains power supply. As another example, the apparatus 2 may comprise a user interface that includes one or more components that enables a user of apparatus 2 (e.g. the subject) to input information, data and/or commands into the apparatus 2 (e.g. switch on, switch off), and/or enables the apparatus 2 to output information or data to the user of the apparatus 2. The user interface can comprise any suitable input component(s), including but not limited to a keyboard, keypad, one or more buttons, switches or dials, a mouse, a track pad, a touchscreen, a stylus, a camera, a microphone, etc., and the user interface can comprise any suitable output component(s), including but not limited to a display screen, one or more lights or light elements, one or more loudspeakers, a vibrating element, etc.

In embodiments where interface circuitry is required to enable communication of measurements/signal from the light sensor 8 to the processing unit 4 and/or communication of a control signal or signals from the processing unit 4 to the light source(s) 10, interface circuitry is present on both sides of the communication link (i.e. the processing unit 4 is associated with first interface circuitry and the light sensor 8 and/or light source(s) 10 are associated with second interface circuitry). The interface circuitry (on both sides of the communication link) is for enabling a data connection and/or a control connection between the processing unit 4 and one or both of the light sensor 8 and light source(s) 10. The connection may be direct or indirect (e.g. via the Internet), and thus the interface circuitry can enable a connection to a network, such as the Internet, via any desirable wired or wireless communication protocol. For example, the interface circuitry can operate using WiFi, Bluetooth, Zigbee, or any cellular communication protocol (including but not limited to Global System for Mobile Communications (GSM), Universal Mobile Telecommunications System (UMTS), Long Term Evolution (LTE), LTE-Advanced, etc.).

In some embodiments, the light sensor 8 and the light source(s) 10 are positioned so that light from the light source(s) 10 is not incident on the light sensor 8. In this way, the signal from the light sensor 8 represents the light from the environment around the subject, and does not include any light emitted by the light source(s) 10 according to the invention. For example, in the spectacles implementation of Fig. 2, the light sensor 8 can be on the frame 22 and face away from the subject, and the light source(s) 10 can be on the frame 22 and face the subject.

In alternative embodiments, the light sensor 8 and the light source(s) 10 are positioned so that light from the light source(s) 10 is incident on the light sensor 8. In this way, the signal from the light sensor 8 represents the light from the environment around the subject and any light emitted by the light source(s) 10 according to the invention. For example, in the spectacles implementation of Fig. 2, the light sensor 8 could be on an arm 26 of the frame 22 and facing generally forward (from the subject's point of view), with the light source(s) 10 being on the frame 22 and facing the subject.

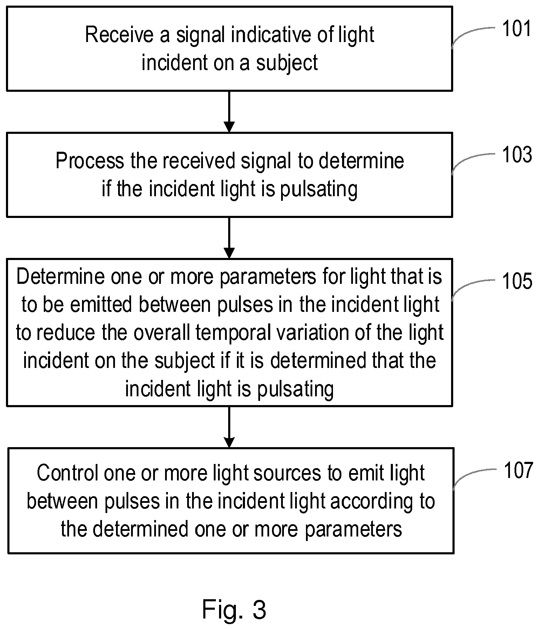

Fig. 3 is a flow chart illustrating an exemplary method of compensating pulsating light according to the invention. The method can be performed by the processing unit 4 in the apparatus 2, in conjunction with a light sensor 8 (whether or not the light sensor 8 is part of the apparatus 2 or separate) and one or more light source(s) 10 (again, whether or not the light source(s) 10 are part of the apparatus 2 or separate).

In some embodiments, computer readable code can be provided that causes the processing unit 4 to perform the method described herein when the processing unit 4 executes the code. The computer readable code can be embodied (i.e. stored) on a computer readable medium, such as in memory unit 6, or any other type of computer program product.

In a first step 101, the processing unit 4 receives a signal indicative of light incident on a subject from the light sensor 8. The light sensor 8 measures the incident light over time, and thus generates a measurement signal indicating the measurement of the incident light at a number of sampling instants. The processing unit 4 preferably receives the signal (or measurement samples) in real-time or near real-time (i.e. with minimal delay from the light sensor 8 measuring the incident light, e.g. with perhaps only a delay due to transmission from the light sensor 8 to the processing unit 4). In some embodiments, the signal indicative of the light is a signal indicative of the intensity of the incident light. In some embodiments, the signal indicative of the light is a signal indicative of the colour of the incident light. In some embodiments, the signal indicative of the light is a signal indicative of the intensity and colour of the incident light (including respective measurements/signals of the intensity of light of different colours (e.g. an intensity measurement/signal of red light, an intensity measurement/signal of green light, an intensity measurement/signal of blue light, etc.)).

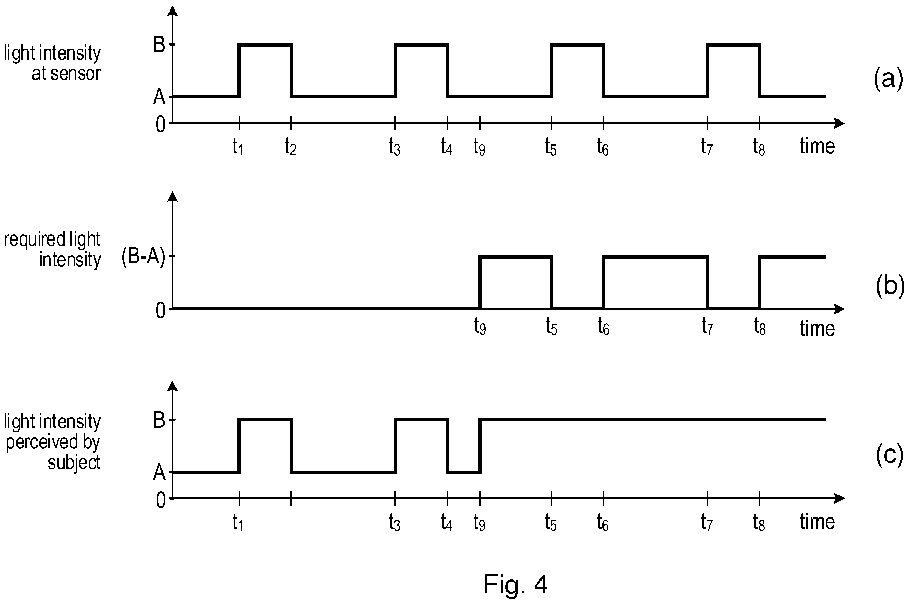

Fig. 4(a) illustrates an exemplary measurement signal that is received from the light sensor 8 over a period of time. In particular, Fig. 4(a) shows a measurement signal for the intensity of the light measured by the light sensor 8, and it can be seen that the light intensity is varying between a first light intensity level A (e.g. representing the ambient light level in the environment around the subject) and a second (higher) light intensity level B over time. Thus, there are pulses of light at the second (higher) light intensity level B between times t₁ and t₂, t₃ and t₄, t₅ and t₆, and t₇ and t₈, with the light intensity being at (lower) level A at all other times.

As the signal is being received in step 101, the processing unit 4 processes the received signal to determine if the incident light is pulsating. This is step 103 in Fig. 3. That is, as the signal is received over time, the processing unit 4 analyses or examines the newly received light measurements in the signal and the previously-received signal for a preceding period of time (e.g. the preceding few seconds, e.g. the preceding 0.1 to 3 seconds) to determine if the light is pulsating. It will be appreciated that the processing unit 4 may need to observe multiple cycles of the light in order to determine that the light is pulsating. For example, based on the measurement signal shown in Fig. 4(a), the processing unit 4 may determine at a time t₉ (which is shortly after time t₄ - the end of the second pulse) that the light is pulsating based on observing two consistent pulses of light between t₁ and t₂ and t₃ and t₄.

In step 103, the processing unit 4 may also determine one or more parameters of the pulsating light. The one or more parameters can be the colour of the pulsating light (if the pulsations are identified from the intensity of the pulsating light), the intensity of the pulsating light (if the pulsations are identified from the changing colour of the light), a pulsation frequency of the light (i.e. how frequently the pulsations occur, e.g. (t₃ - t₁)⁻¹), and a duration of each pulse of light (i.e. how long each pulse lasts, e.g. the time between t₁ and t₂).

In some embodiments of step 103, and particularly (although not exclusively) embodiments that are used to reduce the risk of epileptic seizures, the processing unit 4 can determine in step 103 if the incident light is pulsating, and then determine whether these pulsations are at the same frequency or a similar frequency as brain oscillations of the subject. For example the processing unit 4 can compute the spectrum of the incident light, e.g. via a FFT (Fast Fourier Transform) method, and test for peaks in that spectrum. Any peak at a frequency close to the frequency of a brain oscillation indicates that the incident light is pulsating at the same or a similar frequency as brain oscillations of the subject. As an alternative to computing a FFT, the autocorrelation of the light intensity signal for several time delays τ can be computed. The autocorrelation can be given by: Σ{x(t).x(t-τ)}/Σ{x(t).x(t)}. The autocorrelation can be tested for large values for τ >0, and the value of 1/τ for any large values of τ indicates the frequency of pulsations in the light intensity. Alternatively, in some embodiments of step 103, again particularly (although not exclusively) embodiments that are used to reduce the risk of epileptic seizures, in step 103 the processing unit 4 can determine if the incident light includes pulsations at the same frequency or a similar frequency as brain oscillations of the subject. Thus, in this latter embodiment, the processing unit 4 only aims to identify pulsations having the same or similar frequencies to brain oscillations. The latter embodiment can also be performed using a FFT or autocorrelation as set out above.

If the processing unit 4 determines in step 103 that the light is pulsating, then in step 105, the processing unit 4 determines one or more parameters for light that is to be emitted between the pulses in the incident light by the one or more light source(s) 10 to reduce the overall temporal variation of the light incident on the subject. As noted above, it is desired to compensate pulsating light, and thus the processing unit 4 identifies a profile for light to be emitted by the light source(s) 10 to provide a generally flat envelope of light (e.g. light of a generally constant amplitude or intensity) to the subject. In other words, the processing unit 4 identifies a profile for light to be emitted that is in 'anti-phase' with the pulsations identified in the received signal (i.e. having the opposite phase to the identified pulsations), i.e. between the pulses in the incident light.

Fig. 4(b) shows an exemplary signal representing the light intensity to be output or emitted by the light source(s) 10 in order to provide a generally constant light intensity to the subject. Therefore, the processing unit 4 determines that additional light should be emitted by the light source(s) 10 between the pulses of pulsating light to bring the overall light intensity up to light intensity B (the intensity of the pulsating light) and thus provide a generally constant light intensity. In particular, the processing unit 4 determines that additional light should be emitted by the light source(s) 10 based on the difference between the intensity of the pulsating light and the intensity of the ambient light (i.e. the light to be emitted should have an intensity of (B - A)), in the time periods between each pulse of light at the second intensity level. Thus, as shown in Fig. 4(b), the required light intensity is (B - A) from time t₉ until the expected time of the next pulse (i.e. t₅ based on a determined pulsation frequency), followed by zero additional intensity while the t₅ to t₆ pulse occurs (determined based on the expected duration of the pulse), followed by a light intensity of (B - A) from time t₆ until the expected time of the next pulse (i.e. t₇), followed by zero additional intensity while the t₇ to t₈ pulse occurs, and so on. It can be seen from Fig. 4(b) that the profile of the required light intensity signal is the inverse of the pulsation profile identified in the received measurements.

Thus, in some embodiments, in step 105 the processing unit 4 determines an intensity of the light to be emitted between the pulses in the incident light to match or substantially match the intensity of a component of the incident light that is determined in step 103 to be pulsating. For example, the light sensor 8 can measure the light intensity, and the processing unit 4 can compare this value to a reference intensity (which can be, for example, the average ambient light intensity over the last few minutes), and use the difference between the measured intensity and the average ambient light intensity to generate the control signal (e.g. a driving current) for the light source(s) 10. In some embodiments, in step 105 the processing unit 4 determines a colour of the light to be emitted between the pulses in the incident light to match or substantially match the colour of a component of the incident light that is determined in step 103 to be pulsating. In some embodiments, in step 105 the processing unit 4 determines a pulsation frequency of the light to be emitted between the pulses in the incident light such that the light to be emitted has the opposite phase (anti-phase) to the phase of a component of the incident light that is determined in step 103 to be pulsating. In some embodiments, in step 105 the processing unit 4 determines a duration of each pulse of light to be emitted between the pulses in the incident light based on a time between consecutive pulses of a component of the incident light that is determined in step 103 to be pulsating.

In step 107, the processing unit 4 controls the one or more light sources 10 to emit light between the pulses in the incident light according to the one or more parameters determined in step 105. Step 107 can therefore comprise the processing unit 4 generating and outputting a suitable control signal to the light source(s) 10. In the example of Fig. 4, the processing unit 4 can output a control signal according to Fig. 4(b) so that the light source(s) 10 emit light as shown in Fig. 4(b). This control signal is also referred to herein as a 'masking signal' as the light generated according to this control signal masks the pulsating light by emitting light between the pulses in the incident light. Fig. 4(c) shows the light intensity perceived or observed by the subject while the light source(s) 10 are emitting light. Thus, before time t₉ when the processing unit 4 identifies the pulsations and controls the light source(s) 10 to start emitting light, the subject perceives or sees the pulsing light. After t₉, when the light source(s) 10 are controlled to emit light to 'fill the gaps' between the pulses of light from the environment, the subject perceives a generally constant light intensity (i.e. the temporal variation of the light incident on the subject is reduced). This means that the pulsating light is no longer visible or is as prominent, and the risk of the subject having an epileptic seizure is reduced, and/or the distraction or annoyance caused by the pulsating light is reduced.

The method in Fig. 3 can repeat over time for each new measurement sample or signal, or repeat for each X new measurement samples (in case repeating the method for each measurement sample is too frequent).

It will be appreciated that in embodiments where the light source(s) 10 are arranged so that the light they emit is not incident on the light sensor 8, the light sensor 8 will continue to provide a signal that shows the pulsating light once the light source(s) 10 are being controlled by the processing unit 4 to emit light. In that case, the processing unit 4 can continue to operate as described above to detect the pulsations and determine the one or more parameters of light that should be emitted between the pulses in the incident light to reduce the overall temporal variation of the light incident on the subject. However, in embodiments where the light source(s) 10 are arranged so that the light they emit is incident on the light sensor 8, once the light source(s) 10 are activated by the processing unit 4, the light sensor 8 will provide a signal that includes the contribution of both the pulsating light and the light emitted by the light source(s) 10. In that case, the processing unit 4 will need to adjust the evaluation of the signal to take into account the light contribution from the light source(s) 10 to detect whether the pulsations are continuing (e.g. by subtracting the expected light contribution of the light source(s) 10 from the signal, and then processes the adjusted signal to identify pulsations).

In particular embodiments, in step 105 the processing unit 4 determines parameters for the light to be emitted between the pulses in the incident light such that the emitted light has the same spectral colour and is in anti-phase (i.e. the opposite phase) with the pulsating light.

In some embodiments, this can be achieved using a phase-locked loop (PLL) that uses the received measurement signal as an input. The PLL operates to ensure that the superimposed light intensity (provided by the light source(s) 10) stays in anti-phase/opposite phase with the pulsating light. In the PLL embodiments, the light sensor 8 should preferably be positioned so that the light from the light source(s) 10 is not incident on the light sensor 8. Alternatively, the control signal (e.g. driving current) for the light source(s) 10 can be measured (or known) and used to compensate the sensed light intensity. The PLL embodiments are preferred as the overall light incident on the subject can stay relatively constant.

In some embodiments, the processing unit 4 can perform a quick assessment of the approximate modulation frequency (i.e. the frequency of pulsation), and a masking light signal of approximately the same frequency supplied. In this case the subject perceives pulsating light of a much lower beat/pulse frequency (with this frequency difference being the difference between the frequency of the pulsating light and the modulation frequencies). This is particularly suitable if the frequency of the pulsations is changing over time (e.g. which could be caused by travelling past generally equally-spaced street lamps with a varying speed). Thus, Fig.4(b) shows a masking signal that has the same frequency as the original pulsating light in Fig. 4(a). Fig. 4(c) illustrates a perfect compensation, i.e. with respect to the phase difference between the pulsating light and the masking light. The difference in frequency is 0 Hz, i.e. there is a perfect compensation to produce a constant illimination. If the light emitted by the light source(s) 10 has a slightly different frequency then there will be a period where the two fit quite well, but due to the different frequencies, they will diverge and after a while the pulsating light and the light emitted by the light source(s) 10 will be more synchronous than in anti-phase. Subsequently, the divergence in frequency will lead to the pulsating light and the light emitted by the light source(s) 10 being in anti-phase again. In this case, there is a slow alternation between the light emitted by the light source(s) 10 compensating the pulsating light and amplifying the pulsating light. This slow alternation happens at the difference, or beat, frequency between the pulsating light and the light emitted by the light source(s) 10.

As an alternative to the above embodiments, the light source(s) are driven based on the frequency of the detected pulsations, and a random fluctuation/variation (a "dither" effect) is applied to the detected frequency of the detected pulsations (this embodiment is less complex than locking into anti-phase and perfectly compensating the total intensity).

In other embodiments, the processing unit 4 can implement a feedback loop that uses the measurement signal to determine the control signal for the light source(s) 10.

Generally, the feedback loop is used in steps 103 and 105 to process the received measurement signal and to determine values of the one or more parameters of light that is to be emitted between the pulses in the incident light based on values of the one or more parameters for the pulsating light. In particular the processing unit 4 can implement the feedback loop to determine values for the one or more parameters for the light to be emitted between the pulses in the incident light such that values of the one or more parameters for light subsequently incident on the subject are substantially constant over time.

In some embodiments, the feedback loop operates to determine the control signal such that the measured light intensity stays (generally) constant. In these embodiments the light sensor 8 should measure the light incident on the subject, including the light emitted by the light source(s) 10. In addition, the spectrum (colour) of the pulsating light (or its RGB components) can be measured and the feedback loop used to determine the colour of the light to be emitted by the light source(s) 10. Further, the processing unit 4 may only control the light source(s) 10 in response to detecting the occurrence of pulsating light. This prevents the processing unit 4 from controlling the light source(s) 10 to start emitting light when the subject is in a dark environment (since the processing unit 4 will otherwise drive the light source(s) 10 to meet a certain illumination level, even when no pulsations are present). As in the above embodiments, in some embodiments the processing unit 4 can perform a quick assessment of the approximate modulation frequency (i.e. the frequency of pulsation), and a masking light signal of approximately the same frequency supplied - whereby the subject perceives the much lower beat frequency (the difference between frequency of the pulsations and the modulation frequency of the emitted light).

In some embodiments, the reference light intensity to which the feedback loop adapts the total light intensity (i.e. including the ambient light with the pulsations and the light emitted by the light source(s) 10) can be adaptive or variable. For example the reference light intensity can be the current maximum light intensity according to the light measurements, e.g. the maximum light intensity in a preceding time period (e.g. 10 seconds, a minute, etc.), which is likely to be the intensity of a pulsation when pulsating light is present. Alternatively, the reference light intensity can be a fraction of the maximum light intensity (e.g. 90%), as this can help to stabilise the feedback system.

Thus, according to the above embodiments, the light incident on the subject from the environment and the light source(s) 10 ideally combines into a continuous (in brightness) fluctuating intensity, as depicted in Fig. 4(c). If the compensation provided by the light source(s) 10 is not perfect (i.e. does not exactly match in intensity, frequency and/or phase), undulations in the brightness will remain as a kind of residue. However, the frequency of this residue should be above frequencies that can induce epileptic seizures.

There is therefore provided a method and apparatus for compensating pulsating light.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. An apparatus for compensating pulsating light, the apparatus comprising:
a processing unit configured to:
receive, from a light sensor, a signal indicative of light incident on the subject;
process the received signal to determine if the incident light is pulsating;
determine one or more parameters for light that is to be emitted between pulses in the incident light to reduce the overall temporal variation of the light incident on the subject if it is determined that the incident light is pulsating; and
control one or more light sources to emit light between the pulses in the incident light according to the determined one or more parameters.

2. An apparatus as claimed in claim 1, wherein the one or more parameters comprises a pulsation frequency and phase of the light to be emitted between the pulses in the incident light, and wherein the processing unit is configured to implement a phase locked loop, PLL, to process the received signal and to determine the pulsation frequency and phase of light that is to be emitted between the pulses in the incident light, wherein the received signal of the light incident on the subject is input to the PLL, and the PLL outputs a control signal for the one or more light sources having a pulsation frequency that matches or substantially matches a pulsation frequency of the incident light that is pulsating, and a phase that is opposite to the phase of the incident light that is pulsating.

3. An apparatus as claimed in claim 1, wherein the one or more parameters comprises a pulsation frequency and phase of the light to be emitted between the pulses in the incident light, and wherein the processing unit is configured to determine the one or more parameters by:
estimating a pulsation frequency and phase of the incident light that is pulsating; and
determining the pulsation frequency and phase of light that is to be emitted between the pulses in the incident light such that the frequency of the emitted light matches the estimated pulsation frequency of the incident light that is determined to be pulsating, and determining the phase of the light that is to be emitted between the pulses in the incident light to be opposite to the phase of the incident light that is pulsating.

4. An apparatus as claimed in claim 1, wherein the one or more parameters comprises one or more of a colour of the light, an intensity of the light, a pulsation frequency of the light, and a duration of each pulse of the light.

5. An apparatus as claimed in claim 4, wherein the processing unit is configured to implement a feedback loop to process the received signal and to determine values of the one or more parameters of light that is to be emitted between the pulses in the incident light based on values of the one or more parameters for the light incident on the subject that are pulsating.

6. An apparatus as claimed in claim 5, wherein the feedback loop determines values for the one or more parameters for the light to be emitted between the pulses in the incident light such that values of the one or more parameters for light subsequently incident on the subject are substantially constant over time.

7. An apparatus as claimed in any of claims 1-6, wherein the processing unit is configured to determine if the incident light is pulsating at the same frequency or a similar frequency as brain oscillations of the subject.

8. An apparatus as claimed in any of claims 1-7, wherein the apparatus further comprises the light sensor and/or one or more light sources.

9. An apparatus as claimed in any of claims 1-8, wherein the light sensor is arranged in the apparatus such that the light sensor is close to an eye of the subject when the apparatus is in use by the subject.

10. An apparatus as claimed in any of claims 1-9, wherein the apparatus is in the form of spectacles or other eye or head wear.

11. A computer-implemented method of compensating pulsating light, the method comprising:
receiving, from a light sensor, a signal indicative of light incident on a subject;
processing the received signal to determine if the incident light is pulsating;
determining one or more parameters for light that is to be emitted between pulses in the incident light to reduce the overall temporal variation of the light incident on the subject if it is determined that the incident light is pulsating; and
controlling one or more light sources to emit light between the pulses in the incident light according to the determined one or more parameters.

12. A method as claimed in claim 11, wherein the one or more parameters comprises a pulsation frequency and phase of the light to be emitted between the pulses in the incident light, and wherein the steps of processing and determining comprises implementing a phase locked loop, PLL, to process the received signal and to determine the pulsation frequency and phase of light that is to be emitted between the pulses in the incident light, wherein the received signal of the light incident on the subject is input to the PLL, and the PLL outputs a control signal for the one or more light sources having a pulsation frequency that matches or substantially matches a pulsation frequency of the incident light that is pulsating, and a phase that is opposite to the phase of the incident light that is pulsating.

13. A method as claimed in claim 11, wherein the one or more parameters comprises a pulsation frequency and phase of the light to be emitted between the pulses in the incident light, and wherein the step of determining the one or more parameters comprises:
estimating a pulsation frequency and phase of the incident light that is pulsating; and
determining the pulsation frequency and phase of light that is to be emitted between the pulses in the incident light such that the frequency of the emitted light matches the estimated pulsation frequency of the incident light that is determined to be pulsating, and determining the phase of the light that is to be emitted between the pulses in the incident light to be opposite to the phase of the incident light that is pulsating.

14. A method as claimed in any of claims 11-13, wherein the method further comprises the step of:
emitting light between the pulses in the incident light according to the determined one or more parameters.

15. A computer program product comprising a computer readable medium having computer readable code embodied therein, the computer readable code being configured such that, on execution by a suitable computer or processor, the computer or processor is caused to perform the method of any of claims 11-14.
